(19) Europäisches Patentamt
European Patent Office
Office européen des brevets

(11) Veröffentlichungsnummer : **0 359 706 B1**

# (12) EUROPÄISCHE PATENTSCHRIFT

(45) Veröffentlichungstag der Patentschrift : **21.10.92 Patentblatt 92/43**

(21) Anmeldenummer : **89810645.5**

(22) Anmeldetag : **31.08.89**

(51) Int. Cl.$^5$ : **C07D 493/08,** C07D 493/18, C07H 19/01, A01N 43/90, // (C07D493/00, 321:00, 311:00), (C07D493/18, 321:00, 311:00, 303:00)

## (54) Mikrobizide.

(30) Priorität : **09.09.88 CH 3377/88**

(43) Veröffentlichungstag der Anmeldung : **21.03.90 Patentblatt 90/12**

(45) Bekanntmachung des Hinweises auf die Patenterteilung : **21.10.92 Patentblatt 92/43**

(84) Benannte Vertragsstaaten : **AT BE CH DE ES FR GB GR IT LI LU NL SE**

(56) Entgegenhaltungen : **EP-A- 0 282 455**

(73) Patentinhaber : **Gesellschaft für Biotechnologische Forschung mbH (GBF)**
**Mascheroder Weg 1**
**W-3300 Braunschweig-Stöckheim (DE)**
Patentinhaber : **CIBA-GEIGY AG**
**Klybeckstrasse 141**
**CH-4002 Basel (CH)**

(72) Erfinder : **Sutter, Marius, Dr.**
**Klingental 5**
**CH-4058 Basel (CH)**
Erfinder : **Böhlendorf, Bettina**
**Zimmerstrasse 2**
**W-3300 Braunschweig (DE)**
Erfinder : **Bedorf, Norbert, Dr.**
**Krukenbergstrasse 4**
**W-3308 Königslutter (DE)**
Erfinder : **Höfle, Gerhard, Prof. Dr.**
**Alter Weg 12a**
**W-3300 Braunschweig (DE)**

EP 0 359 706 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft eine macrocyclische Verbindung der Formel I, ein Verfahren zu ihrer Herstellung und ihre Verwendung zur Bekämpfung von Pflanzenkrankheiten sowie pflanzenmikrobizide Mittel, die diese Verbindung als Wirkstoff enthalten.

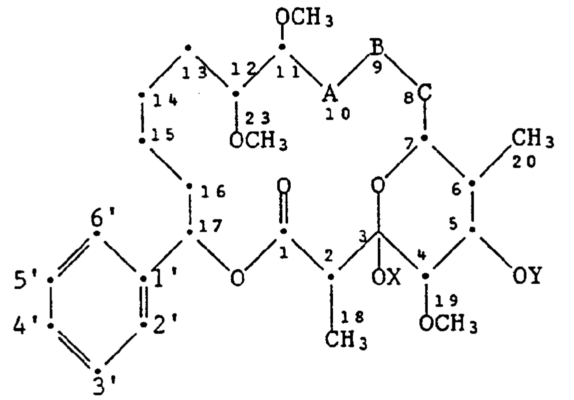

(I)

In dieser Formel bedeutet
X = Wasserstoff oder Methyl,
Y = Wasserstoff, -CHO, -$COCH_3$, Benzyl, Methyl oder eine Silyl-Gruppe, und
A-B-C folgende $C_3$-Kettenglieder:

a) 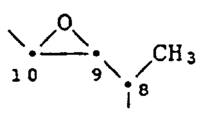 b) 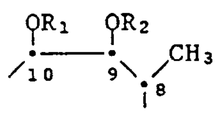 c) 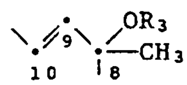 d) 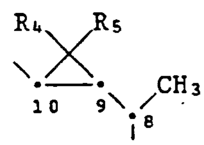

worin
$R_1$, $R_2$ und $R_3$ unabhängig voneinander Wasserstoff, Methyl, -CHO, -$COCH_3$, oder eine Silyl-Gruppe darstellen, und
$R_4$ und $R_5$ Wasserstoff, Halogen oder -COOAlkyl($C_1$-$C_3$) bedeuten.

Unter Silyl-Gruppen werden solche Reste verstanden, die üblicherweise als Silyl-Schutzgruppen an z.B. Hydroxy-Gruppen bei Naturstoffsynthesen verwendet werden. Genannt seien beispielsweise Trimethylsilyl, Diphenyl-tert.butylsilyl, bis(Isopropyl)-methylsilyl, Triphenylsilyl usw. und insbesondere tert.Butyl-dimethylsilyl.

Unter $C_1$-$C_3$-Alkyl werden Methyl, Ethyl, Propyl und Isopropyl verstanden.

Die Verbindungen der Formel I leiten sich ab von der "Soraphen A" genannten Grundstruktur einer neuen natürlichen macrocyclischen Verbindung der Formel

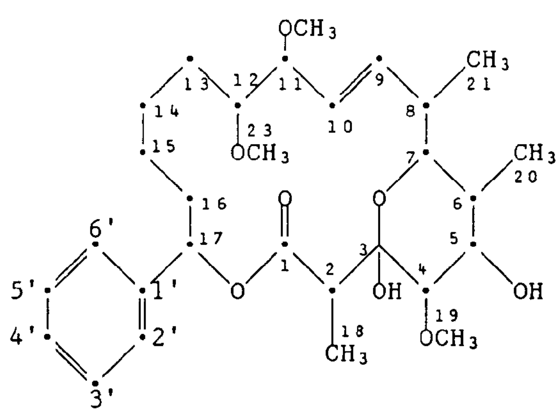

Soraphen A

Aufgrund seiner physikochemischen Daten wird angenommen, dass diesem Präparat folgende Konfiguration zukommt:

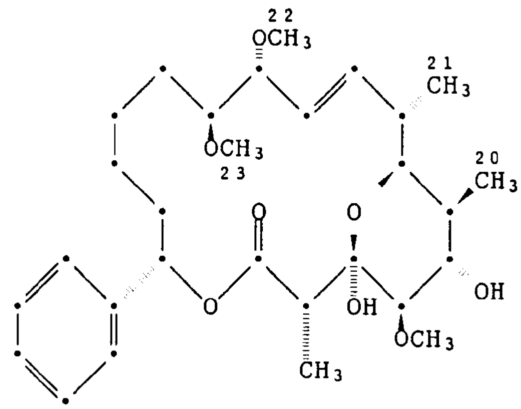

Soraphen A wird durch mikrobiologische Kultivierung eines Sorangium (Polyangium) cellulosum-Stammes "So ce 26" gewonnen. Dieser Stamm wurde am 5. März 1987 bei der "National Collection of Industrial and Marine Bacteria (NCIB)", Torry Research Station, Aberdeen, Grossbritannien, unter der Nummer NCIB 12 411 gemäss Budapester Vertrag hinterlegt. Sorangium cellulosum gehört zur Ordnung der Myxobacterales, Unterordnung Sorangineae, Familie Polyangiaceae.

"So ce 26" selbst oder Mutanten oder Rekombinanten sind Gegenstand der europäischen Patentanmeldung EP-A-0282 455. Der Stamm lässt sich nach üblichen biologischen Methoden, z.B. in Schüttelkulturen oder in Fermentoren mit Nährmedien bei 10-35°C und einem pH-Wert von 6-8, kultivieren. Die Fermentationstemperatur beträgt im Regelfall 10-35°C und liegt vorzugsweise bei etwa 30-32°C, der pH-Wert beträgt 6-8, bevorzugt 7,4. Der Prozess verläuft aerob und unter sterilen Bedingungen. Die Bedingungen zur Kultivierung des Mikroorganismus werden per Referenz zur EP-A-0282 455 in die vorliegende Beschreibung eingeführt.

Das erfindungsgemässe Verfahren zur Herstellung einer Verbindung der Formel I aus Soraphen A ist gekennzeichnet wahlweise und unabhängig von der Reihenfolge

a) durch Carben-Anlagerung, Selendioxid-Oxidation, Osmiumtetroxid-Oxidation oder Epoxidierung in 9,10-Stellung unter gegebenenfalls weiterer Ringöffnung des gebildeten Epoxids und/oder, sofern gewünscht,

b) durch Methylierung der 3-OH-Gruppe, und/oder, sofern gewünscht,

c) durch Formylierung, Acetylierung, Methylierung, Benzylierung bzw. Silylierung der 5-OH-Gruppe und/oder der neu gebildeten OH-gruppen in 8-, 9- oder 10-Stellung.

Die Wahl der Reaktionsschritte hängt von der durchzuführenden Reaktion ab.

Soll die 9,10-Doppelbindung in der Formel I in ein 9,10-Epoxid gemäss Definition a) verwandelt werden, so lässt sich dies durch Oxidation von Soraphen A oder von einem in 5-Stellung geschützten (möglicherweise auch in 3-Stellung geschützten) Soraphen A-Derivat im Temperaturbereich von -30°C bis +100°C, bevorzugt -10°C bis +30°C, und zweckmässig bei 0° bis 10°C mit $H_2O_2$ oder einer Persäure wie m-Chlorperbenzoesäure oder Peressigsäure in inerten Lösungsmitteln wie Dichlormethan, Toluol oder Benzol, gegebenenfalls in Gegenwart einer Base wie $NaHCO_3$ oder $Na_2HPO_4$ erzielen. Die Oxidation lässt sich auch mit tert. Butylhydroperoxid in Gegenwart von Katalysatoren wie Vanadium(V)-Komplexen, z.B. ausgehend vom Vanadyl(IV)-acetylacetonat, oder Mo-(VI)-Komplexen, z.B. ausgehend vom Molybdänhexacarbonyl in inerten Lösungsmitteln wie trockenen Kohlenwasserstoffen (z.B. Benzol, Toluol, Xylol) durchführen.

Sofern das 9,10-Epoxid in ein 9,10-Diol vom Typ b) umgewandelt werden soll, lässt sich das Epoxid mit milden Lewis-Säuren wie $ZnCl_2$, $ZnBr_2$ oder $TiCl_4$ in Gegenwart von Wasser öffnen.

Vorteilhaft lassen sich 9,10-Diole aus dem Olefin durch Reaktion mit Osmiumtetroxid bei 0° bis 100°C, bevorzugt bei Raumtemperatur erhalten. Als Lösungsmittel dienen beispielsweise Aether, Tetrahydrofuran oder Aceton gegebenenfalls unter Zusatz von Pyridin.

Sofern Verbindungen des Typs c) mit einer Sauerstoff-Funktion in 8-Stellung hergestellt werden sollen, so lässt sich die 9,10-Doppelbindung des "Soraphen " mit Selendioxid in Lösungsmitteln wie Essigsäure, Acetanhydrid, Dioxan oder Alkoholen, gegebenenfalls unter Zusatz von Wasser, bei 0° bis 100°C oxidieren.

Die gemäss Definition unter d) genannten Cyclopropanderivate können durch Umsetzung der entsprechenden Olefine mit Carbenen erhalten werden. (Houben-Weyl, "Methoden der organischen Chemie", Band 413, S. 98ff)

Carbene lassen sich in literaturbekannter Weise, beispielsweise ausgehend von Diazomethan herstellen, das pyrolytisch, photolytisch oder katalytisch (z.B. in Gegenwart von Kupfer, Kupfer(II)sulfat, Kupfer(I)chlorid, Platin(IV)chlorid, Zinkjodid) zum [$H_2C$l] verwandelt wird. Ein weiterer Zugang zu Carbenen besteht in der Methode nach Simmon-Smith, bei der Methylendijodid mit einem Zink-Kupfer-Paar zum [$H_2C$l] reagiert.

Halogencarbene ($R_4$ und $R_5$ = Halogen) lassen sich aus entsprechend substituierten Methanderivaten durch Behandlung mit Basen herstellen. Als Carbenbildner kommen z.B. Chloroform, Bromoform, Dichlormethan in Frage. Basische Komponenten sind beispielsweise KOH, Alkalimetall-Alkoholate wie K-tert. Butylat oder Alkyllithium wie n-Butyllithium. Halogencarbene lassen sich auch aus entsprechenden Salzen von perhalogenierten Essigsäuren durch Erwärmen auf 50 bis 300°C herstellen.

Alkoxycarbonylcarbene ($R_4$ und $R_5$ = -COOAlkyl) sind aus entsprechenden Diazoessigsäureestern durch katalytische Zersetzung erhältlich. Als Katalysatoren können beispielsweise metallisches Kupfer oder Kupfersalze wie $CuSO_4$, oder Rhodiumsalze wie Rh(II)-Acetat eingesetzt werden.

Als Lösungsmittel kann in allen diesen Fällen der Carbenbildner allein oder im Gemisch mit inerten Lösungsmitteln wie Ethern (Diethylether, Diglyme, Dioxan, Tetrahydrofuran) oder Kohlenwasserstoffen (z.B. Petrolether) dienen.

Die Reaktionstemperaturen für Gewinnung und Einsatz entsprechender Carbene liegen zwischen -50° und +300°C.

Methylierungen einer bereits existierenden Hydroxygruppe im "Soraphen A" lassen sich zweckmässigerweise mit Methyljodid in Dimethylsulfoxid bei Raumtemperatur unter Zusatz einer Base wie KOH durchführen. Benzylierungen werden vorteilhaft mit Benzylbromid durchgeführt.

Durch übliche Acylierung einer OH-Gruppe mit der entsprechenden Carbonsäure bzw. mit einem entsprechenden Acylhalogenid oder Acylanhydrid oder Silylierung durch Reaktion einer OH-Gruppe mit dem entsprechend substituierten Silan-Derivat der Formel

$$X-Si\lessdot$$

das an den freien Bindungen Alkyl, Phenyl oder Benzyl tragen kann, werden alle Formyl-, Acetyl- oder Silyl-Derivate gemäss Definition erhalten, wobei der Begriff Acylhalogenid für Acylchlorid oder Acylbromid steht und wobei X eine Silylabgangsgruppe bedeutet. Zu den Silylabgangsgruppen X zählen beispielsweise Bromid, Chlorid, Trifluormethansulfonat.

O-Acylierungen und O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0°C bis 80°C, bevorzugt bei 10°C bis 50°C ab. Vorzugsweise wird eine organische Base zugegeben. Genannt seien beispielsweise tertiäre Amine wie Triethylamin, Triethylendiamin, Triazol und bevorzugt Pyridin, Diisopropylamin, 4-Dimethylaminopyridin, Imidazol oder 1,8-Diazabicyclo[5.4.0]-undec-7-en (DBU).

Geeignete Lösungsmittel sind z.B.: Ether und etherartige Verbindungen wie Dialkylether (Diethylether, Diisopropylether, tert.-Butylmethylether, Dimethoxiethan, Dioxan, Tetrahydrofuran oder Anisol); halogenierte Kohlenwasserstoffe wie Chlorbenzol, Methylenchlorid, Ethylenchlorid, Chloroform, Tetrachlorkohlenstoff oder Tetrachlorethylen; Dimethylformamid (=DMF) oder Sulfoxide wie Dimethylsulfoxid, wobei auch aromatische oder aliphatische Kohlenwasserstoffe, wie Benzol, Toluol, Xylole, Petrolether, Ligroin oder Cyclohexan anwesend sein können. In manchen Fällen kann es von Vorteil sein, wenn die Reaktionen unter Schutzgasatmosphäre (z.B. Argon, Helium oder Stickstoff) und/oder in absoluten Lösungsmitteln durchgeführt werden.

Werden Säurehalogenide oder Säureanhydride zur Acylierung eingesetzt, so erweist sich der Zusatz eines Neutralisationsmittels als vorteilhaft. Tertiäre Amine wie Trialkylamine, Pyridin oder Pyridinbasen wie 4-Dimethylaminopyridin sind zweckmässige Reagenzien.

Sind im Molekül oder im Reaktanden störende funktionelle Gruppen wie OH, so können diese, wie bereits oben erwähnt, durch Acetylierung oder Einführung anderer Schutzgruppen, wie auch der Silylgruppe, einleitend maskiert werden [T.W. Green "Protective Groups in Organic Synthesis", J. Wiley & Sons, 1981 (New York)].

Sofern gewünscht, lassen sich Schutzgruppen, wie Acylgruppen, durch milde Verseifung (in der Regel mit z.B. $NH_3$/Methanol) abspalten. Als Lösungsmittel kommen bei diesem Teilschritt insbesondere aprotische Vertreter wie Dichlormethan, Acetonitril, Benzol, Toluol, Nitromethan, Dioxan, THF, Ethylenglykoldimethylether in Frage; Diethylether ist besonders geeignet.

Silylgruppen lassen sich durch Behandlung mit Säure oder Fluorid-Ionen abspalten, bevorzugt Fluorwasserstoffsäure in Acetonitril oder Tetrabutylammoniumfluorid in Tetrahydrofuran (=THF).

Gewünschtenfalls können die Endprodukte auf übliche Art und Weise gereinigt werden, z.B. durch Waschen, Digerieren, Extraktion, Umkristallisation oder Chromatographie.

Die beschriebenen Herstellungsverfahren einschliesslich aller Teilschritte zur Gewinnung von Verbindungen der Formel I in allen möglichen stereoisomeren Formen sind ein Bestandteil der vorliegenden Erfindung.

Eine wichtige Gruppe von Verbindungen im Umfang der Formel I ist die, worin A-B-C wahlweise

a) 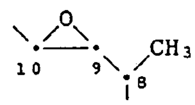 oder b) 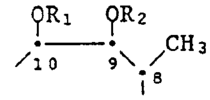

darstellen, wobei $R_1$ und $R_2$ unabhängig voneinander Wasserstoff, Methyl, -CHO oder $-COCH_3$ sind, während X und Y die genannte Bedeutung haben. In dieser Gruppe sind jene bevorzugt, worin X Wasserstoff bedeutet.

Weiterhin sind Verbindungen der Formel I wichtig, worin A-B-C wahlweise

a) 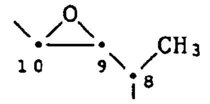 oder b) 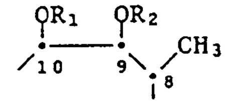

darstellen, X Wasserstoff ist und Y, $R_1$ und $R_2$ unabhängig voneinander Wasserstoff, Methyl, -CHO, $-COCH_3$ oder eine Silyl-Schutzgruppe bedeuten.

Innerhalb der Untergruppe Ic sind diejenigen bevorzugt, worin Y Wasserstoff, Methyl, -CHO, $-COCH_3$ oder eine Silyl-Schutzgruppe und $R_1$ und $R_2$ die gleiche Bedeutung Wasserstoff, Methyl oder Formyl haben.

Unter den bevorzugten Einzelverbindungen sind die folgenden zu nennen:

9,10-Epoxi-Soraphen A,
9,10-Dihydroxy-Soraphen A,
5,9,10-Triformyl-Soraphen A,
5,9,10-Triacetyl-Soraphen A,
9,10-Epoxi-Soraphen A-5-acetat.

Es ist zu beachten, dass die macrocyclischen Soraphene der Formel I normalerweise in der angegebenen Hemiacetalform vorliegen, diese Form jedoch eine reversible Ringöffnung nach dem Schema

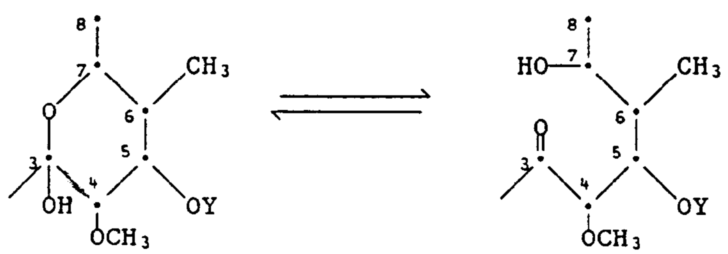

erfahren kann. Je nach Herstellungs- bzw. nach Aufarbeitungsmethodik fallen, abhängig vom pH-Wert und vom Lösungsmittel, die Soraphene in der einen oder anderen Form oder als Gemisch beider Formen an. Charakteristisch für die Ringöffnung ist die Verschiebung des $^{13}C$-NMR-Signals in der 3-Position und die der $^{1}H$-NMR-Signale in bestimmten anderen Positionen. Beim Soraphen A werden beispielsweise folgende Veränderungen beobachtet:

$^{13}C$-NMR($CDCL_3$, $\delta$ in ppm) 99,5 $\rightarrow$203,1(3-C). $^{1}H$-NMR($CDCl_3$, $\delta$ in ppm): 3,14$\rightarrow$3,72(2-H); 3,18$\rightarrow$4,5(4-H); 3,83$\rightarrow$3,16 (7-H); 5,86$\rightarrow$5,7 (17-H). Aehnliche Verschiebungen werden auch bei den hierin beschriebenen Soraphen-Derivaten der Formel I beobachtet. Die Formel I vorliegender Erfindung umfasst grundsätzlich sowohl die bei niedrigen pH-Werten bevorzugte 3-Hemiacetalform wie auch die geöffnete 3-Keto-7-hydroxyform.

Es wurde gefunden, dass Verbindungen der Formel I ein für praktische Bedürfnisse sehr günstiges biozides Spektrum gegen phytopathogene Mikroorganismen, insbesondere gegen Pilze, aufweisen. Sie besitzen sehr vorteilhafte kurative, systemische und insbesondere präventive Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen verschont bleiben.

Als Mikrobizide sind die Wirkstoffe der Formel I z.B. gegen die den folgenden Klassen angehörenden phytopathogenen Pilze wirksam: Fungi imperfecti (z.B. insbesondere Botrytis, ferner Pyricularia, Helminthosporium, Fusarium, Septoria, Cercospora und Alternaria); Basidiomyceten (z.B. Rhizoctonia,

Hemileia, Puccinia). Darüber hinaus wirken sie gegen die Klasse der Ascomyceten (z.B. insbesondere Venturia und Erysiphe, ferner Podosphaera, Monilinia, Uncinula) und der Oomyceten (z.B. Phytophthora, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I in irgendeiner der möglichen stereoisomeren Formen enthalten, insbesondere pflanzenschützende Mittel, sowie deren Verwendung auf dem Agrarsektor oder verwandten Gebieten.

Eingeschlossen ist auch ein Verfahren zur Behandlung von Pflanzen, das sich durch Applikation der neuen Verbindungen der Formel I bzw. der entsprechenden neuen Mittel auszeichnet.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Ananas, Zuckerrohr, Tee, Pfeffer, Weinreben, Hopfen, Bananen- und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere des Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den boden einbringt, z.B. in Form von Granulat (Bodenapplikation). Verbindungen der Formel I können auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden siw zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 10 g bis 500 g Aktivsubstanz (AS) pro Hektar, bevorzugt bei 50 g bis 200 g AS/ha.

Die Formulierungen d.h. die den Wirkstoff der Formel I und einen festen oder flüssigen Zusatzstoff enthaltenden Mittel werden in bekannter Weise hergestellt.

Als Lösungsmittel kommen in Frage: Aromatische und aliphatische Kohlenwasserstoffe, wie z.B. Xylolgemische, Cyclohexan oder Paraffine; dann Alkohole und Glykole sowie deren Ether und Ester, wie Ethanol, Ethylenglykol, Ethylenglykolmonomethyl- oder -ethylether, Essigsäureester; Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien, z.B. Calcit oder Sand, in Frage.

Darüber hinaus kann eine Vielzahl von vorgranulierten Materialien anorganischer Natur wie insbesondere Dolomit oder zerkleinerte Pflanzenrückstände verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Häufiger werden jedoch sog. synthetische Tenside verwendet, insbesondere Alkansulfonate, Fettalkoholsulfate, sulfonierte Benzimidazolderivate oder Alkylsulfonate.

Als nichtionische Tenside kommen Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Als weitere Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 95 % Wirkstoff der Formel I, 99,9 bis 5 % eines festen oder flüssigen Zusatzstoffes und 0 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung.

## 1. Herstellungsbeispiele

### H-1. Herstellung von 9,10-Dihydroxi-Soraphen A (Verb. 1)

21 mg Soraphen A werden unter leichtem Rühren in 10 ml Diethylether mit 100 mg Osmiumtetroxid und 2 ml Pyridin versetzt und 8 Tage bei Raumtemperatur stehen gelassen. Die überstehende Lösung wird abpipettiert, und der entstandene Niederschlag wird in 3 ml Dichlormethan gelöst und durch vorsichtiges Zutropfen von Hexan von neuem ausgefällt. Nach Entfernen des Lösungsmittels wird der Niederschlag getrocknet.

Dieser wird mit einer Lösung von 1,8 g $NaHSO_3$ in 30 ml Wasser und 20 ml Pyridin versetzt und 25 min. bei Raumtemperatur gerührt. Danach wird die Lösung dreimal mit je 50 ml Dichlormethan extrahiert. Die vereinigte organische Phase wird einmal mit 30 ml gesättigter NaCl-Lösung gewaschen, über $Na_2SO_4$ getrocknet und eingeengt. Das zurückbleibende Oel wird an Kieselgel mit Ethylacetat/Hexan (3:1) als Laufmittel gereinigt: 5,1 mg Endprodukt.

$R_f$ (Hexan/Ethylacetat = 1:2) : 0,12

MS: $M^+$ = 554

$^1$H-NMR (Deutero-Aceton): 5,85 ppm (t,J = 7Hz, H-$C^{17}$)

250 MHZ 3,46 ppm, 3,41 ppm, 3,39 ppm } (3s) (3-$OCH_3$)

$^{13}$C-NMR (Deutero-Aceton): 69,2; 70,3; 73,9; 74,7; 75,2; 77,1; 81,7; 84,1(8d); 101,1(s).

### H-2. Herstellung von 5-tert Butyldimethylsilyl-9,10-epoxy-Soraphen A (Verb. 26)

1,0 g Soraphen A in 5 ml DMF werden bei Raumtemperatur mit 1,1 g Imidazol und 1,6 g

tert.Butyldimethylsilylchlorid versetzt und während 3 Tagen gerührt. Man giesst auf Diethylether, wäscht mit 1 N Chlorwasserstoffsäure, dann mit gesättigter Natriumhydrogencarbonatlösung und gesättigter Kochsalzlösung, trocknet über Natriumsulfat, filtriert und entfernt das Lösungsmittel. Chromatographie liefert 0,84 g 5-tert.Butyldimethylsilyl-Soraphen A.

0,51 g dieser Verbindung werden in 5 ml Methylenchlorid gelöst und mit 0,82 g m-Chlorperbenzoesäure versetzt. Nach beendeter Reaktion wird mit Ethylacetat verdünnt und mit gesättigter Natriumhydrogencarbonatlösung gewaschen, über Natriumsulfat getrocknet und das Lösungsmittel entfernt. Chromatographie liefert 0,34 g Produkt.

H-3. Herstellung von 9,10-Epoxy-Soraphen A (Verb. 7)

10 mg der Verbindung Nr. 26 werden mit 1 ml 1N Tetrabutylammoniumfluorid-Lösung in THF versetzt und 20 Minuten bei Raumtemperatur gerührt. Das Gemisch wird in Essigsäureethylester aufgenommen, mit gesättigter Kochsalzlösung gewaschen, über Natriumsulfat getrocknet und eingeengt. Chromatographie liefert 2,7 mg 9,10-Epoxy-Soraphen A.

H-4. Herstellung von 5-tert.Butyldimethylsilyl-9,10-dihydroxy-Soraphen A (Verb.Nr. 35)

1,11 g der Verbindung Nr. 26 werden in 2 ml Benzol gelöst und mit 3,8 g Zinkbromid versetzt. Die Suspension wird über Nacht bei Raumtemperatur gerührt, filtriert und eingeengt. Chromatographie liefert 0,34 g der Verbindung Nr. 35, aus der man durch Entsilylierung mit Fluorid das zu Verbindung 1 epimere 9,10-Dihydroxi-Soraphen A erhält, bei dem beide OH-Gruppen auf gleicher Seite der Molekülebene stehen.

Tabelle 1:

| Verb. Nr. | $R_f$(Lsgm.) | MS(FD) | $^1$H-NMR (Deuteroaceton) |
|---|---|---|---|
| 1 | 0,27(2) | | 5,85(H-17) 5,60(OH) 5,06(OH) 4,24 |
| 7 | 0,32(1) | 537($M^+$+H) | 6,02(H-17) 5,76(OH) 5,36(OH) 4,36(H-5) 4,08(H-7) |
| 9 | 0,66(1) | 579($M^+$+H) | 5,88(H-17) 5,11(OH) 4,21(H-7) |
| 17 | 0,30(2) | | 5,83(H-17) 5,17(OH) 4,80 4,72 |
| 26 | 0,27(3) | 651($M^+$+H) | 6,02(H-17) 5,55(OH) 4,45(H-5) 4,08(H-7) |
| 27 | 0,38(2) | 551($M^+$+H) | 6,03(H-17) 5,10(OH) 3,90(H-7) |
| 28 | 0,45(2) | 668($M^+$)* | 5,86(H-17) 5,34(OH) 4,34 4,18 |
| 31 | 0,51(1) | 720($M^+$)* | 6,10(H-17) 5,40(HO) 5,22(3 OH) 4,48(H-7) 4,32(H-5) |
| 33 | 0,40(1) | 668($M^+$)* | 6,10(H-17) 5,50(OH) 4,74(H-7) 4,42(H-5) 4,12 |

(1) Ethylacetat/Hexan 1:1
(2) Ethylacetat/Hexan 3:1
(3) Diethylether/Hexan 1:1
* = CI

Auf diese Art oder nach einer der weiter oben angegebenen Methoden lassen sich die in der folgenden Tabelle genannten Soraphen-A-Derivate gewinnen.

Tabelle 2

| Nr. | X | Y | A B C |
|---|---|---|---|
| 1 | H | H | 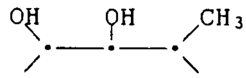 |
| 2 | H | CHO | 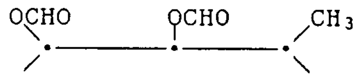 |
| 3 | H | $COCH_3$ | 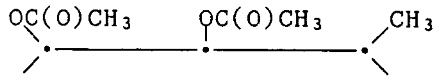 |
| 4 | $CH_3$ | $CH_3$ | 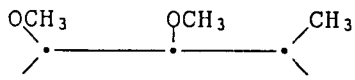 |
| 5 | H | $Si(CH_3)_3$ | 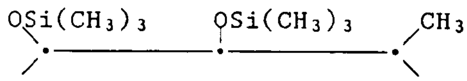 |
| 6 | H | 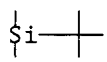 | 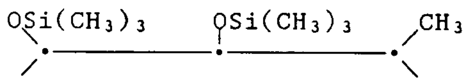 |
| 7 | H | H | 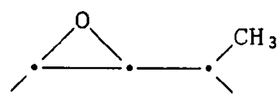 |
| 8 | $CH_3$ | $CH_3$ | 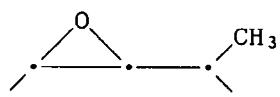 |
| 9 | H | $COCH_3$ | 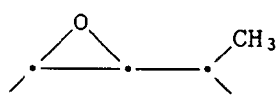 |
| 10 | H | $COCH_3$ | 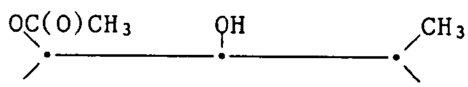 |
| 11 | H | $COCH_3$ | 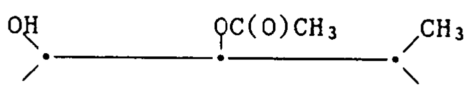 |
| 12 | H | $COCH_3$ | 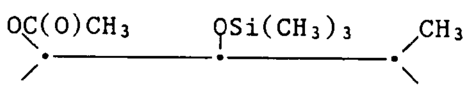 |
| 13 | H | H | 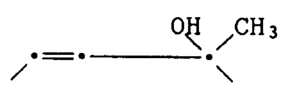 |
| 14 | H | H | 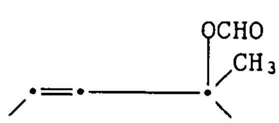 |

Tabelle 2 (Fortsetzung)

| Nr. | X | Y | A B C |
|---|---|---|---|
| 15 | $CH_3$ | $CH_3$ | 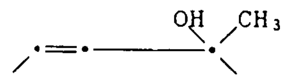 |
| 16 | H | $COCH_3$ | 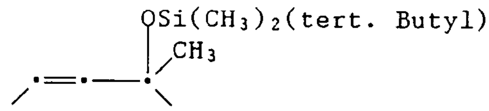 |
| 17 | H | O Benzyl | 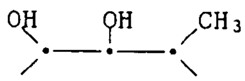 |
| 18 | H | H | 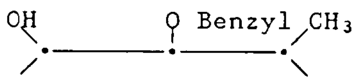 |
| 19 | H | H | 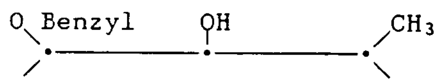 |
| 20 | H | H | 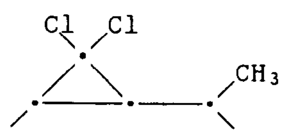 |
| 21 | H | H | 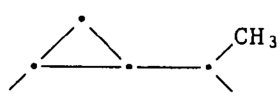 |
| 22 | H | $COCH_3$ | 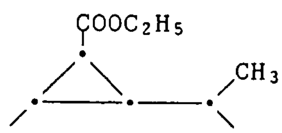 |
| 23 | H | H | 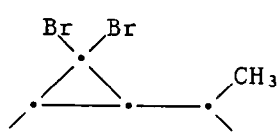 |
| 24 | H | H | 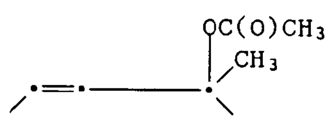 |
| 25 | H | H | 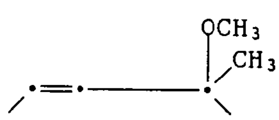 |

Tabelle 2 (Fortsetzung)

| Nr. | X | Y | A B C |
|---|---|---|---|
| 26 | H | 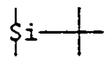 | 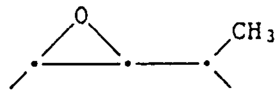 |
| 27 | H | $CH_3$ | 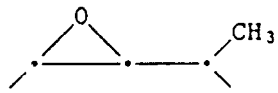 |
| 28 | H | 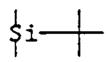 | 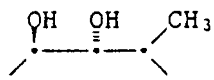 bzw. 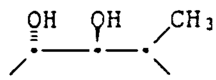 |
| 29 | H | $CH_3$ | 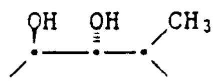 bzw. 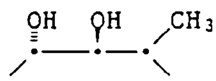 |
| 30 | H | 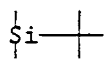 | 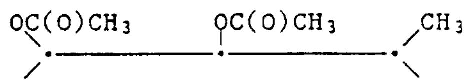 |
| 31 | H | 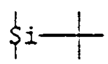 | 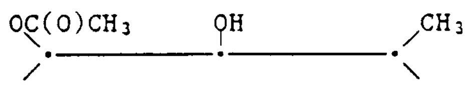 |
| 32 | H | 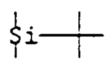 | 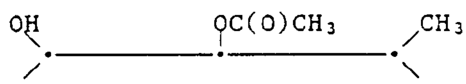 |
| 33 | H | 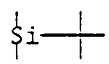 | 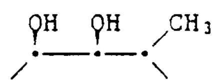 bzw. 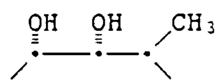 |
| 34 | H | $CH_3$ | 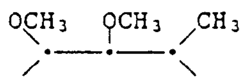 |

Tabelle 2 (Fortsetzung)

| Nr. | X | Y | A B C |
|---|---|---|---|
| 35 | H | -CHO | 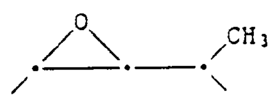 |

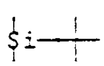 = tert.Butyl-dimethylsilyl

2. Formulierungsbeispiele für den Wirkstoff der Formel I (% = Gewichtsprozent) ["Wirkstoff" bedeutet im folgenden einen Wirkstoff aus vorstehender Tabelle 2]

| 2.1 Emulsions-Konzentrate | a) | b) | c) |
|---|---|---|---|
| Wirkstoff | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenol-polyethylenglykol-ether (30 Mol Ethylenoxid) | - | 12 % | 4 % |
| Cyclohexanon | - | 15 % | 20 % |
| Xylolgemisch | 65 % | 25 % | 20 % |

Aus solchen Konzentration können durch Verdünnen Wasser Emulsionen jeder gewünschten Konzentration hergestellt werden.

| 2.2 Lösungen | a) | b) | c) | d) |
|---|---|---|---|---|
| Wirkstoff | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

(MG = Molekulargewicht)

Die Lösungen sind zur Anwendung in Form kleinster Tropfen geeignet.

| 2.3 Granulate | a) | b) |
|---|---|---|
| Wirkstoff | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel. Diese lassen sich durch weiteren Zusatz der drei Trägerstoffe auf anwendungsfertige Stäube mit 0,001 % Wirkstoff vermahlen.

| 2.5 Spritzpulver | a) | b) | c) |
|---|---|---|---|
| Wirkstoff | 25 % | 50 % | 75 % |
| Na-Ligninsulfonat | 5 % | 5 % | - % |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - % |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.6 Umhüllungs-Granulat | |
|---|---|
| Wirkstoff | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.7 Suspensions-Konzentrat

| | |
|---|---|
| Wirkstoff | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspension jeder gewünschten Konzentration hergestellt werden können.

3. Biologische Beispiele an Pflanzen

(Im folgenden bedeutet "Wirkstoff" ohne weiteren Hinweis ein Präparat aus der Tabelle 2.)

Beispiel 3.1: Wirkung gegen Puccinia graminis auf Weizen

a) Residual-protektive Wirkung

Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

b) Systemische Wirkung

Zu Weizenpflanzen wird 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Der Pilzbefall wurde in beiden Versuchen vollständig durch den Wirkstoff der Formel I gehemmt. Mit den Verbindungen Nr. 1, 2, 7 und 34 wurde der Pilzbefall im Versuch a) auch noch in einer Konzentration von 0,006 % unterbunden (0-5 %).

Unbehandelte aber infizierte Kontrollpflanzen wiesen dagegen einen Puccinia-Befall von 100 % auf.

Beispiel 3.2: Wirkung gegen Phytophthora auf Tomatenpflanzen

a) Residual-protektive Wirkung

Tomatenpflanzen wurden nach 3-wöchiger Anzucht mit einer aus Spritzpulver des Wirkstoffes hergestellten Spritzbrühe (0,02 % Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20°C.

b) Systemische Wirkung

Zu Tomatenpflanzen wurde nach 3-wöchiger Anzucht eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % relativer Luftfeuchtigkeit und 20°C.

In beiden Versuchen wurde bei der Answertung kein Pilzbefall beobachtet.

Beispiel 3.3: Wirkung gegen Plasmopara viticola auf Reben Residual-protektive Wirkung

Im 4-5 Blattstadium werden Rebensämlinge mit einer aus Spritzpulver des Wirkstoffes hergestellten Spritzbrühe (0,006 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20°C wird der Pilzbefall beurteilt.

Im Gegensatz zu den unbehandelten, aber infizierten Kontrollpflanzen mit 100 % Pilzbefall waren die mit dem Wirkstoff I behandelten Pflanzen befallsfrei.

Beispiel 3.4: Wirkung gegen Cercospora arachidicola auf Erdnusspflanzen Residual protektive Wirkung

10-15 cm hohe Erdnusspflanzen werden mit einer aus Spritzpulver der Wirksubstanz hergestellten Spritzbrühe (0,006 % Aktivsubstanz) besprüht und 48 Stunden später mit einer Konidiensuspension des Pilzes infiziert. Die infizierten Pflanzen werden während 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Die mit dem Wirkstoff I behandelten Pflanzen waren ohne Befall. Mit den Verbindungen Nr. 1, 2, 7, 27, 29, 34 und 35 wurde der Pilzbefall auch noch in einer Konzentration von 0,002 % Wirkstoff vollständig gehemmt (0-5 % Befall). Unbehandelte aber infizierte Kontrollpflanzen wiesen dagegen einen Cercospora-Befall von 100 % auf.

Beispiel 3.5: Wirkung gegen Venturia inaequalis auf Apfeltrieben Residual-protektive Wirkung

Apfelstecklinge mit 10-20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24°C aufgestellt. Der Schorfbefall wird 15 Tage nach der Infektion beurteilt.

Die mit dem Wirkstoff I behandelten Stecklinge waren befallsfrei.

Beispiel 3.6: Wirkung gegen Botrytis cinerea an Apfelfrüchten Residual-protektive Wirkung

Künstlich verletzte Aepfel werden behandelt, indem eine aus Spritzpulver der Wirksubstanz hergestellte Spritzbrühe (0,006 % Aktivsubstanz) auf die Verletzungsstellen aufgetropft wird. Die behandelten Früchte werden anschliessend mit einer Sporensuspension des Pilzes inokuliert und während einer Woche bei hoher Luftfeuchtigkeit und ca. 20°C inkubiert. Bei der Auswertung werden die angefaulten Verletzungsstellen gezählt und daraus die fungizide Wirkung der Testsubstanz abgeleitet.

Der Wirkstoff I hemmte den Pilzwuchs vollständig. Mit den Wirkstoffen Nr. 1, 2, 3, 4, 7, 8, 9, 17, 26, 27, 28, 29, 30 und 33-35 wurde der Pilzbefall auch noch in einer Konzentration von 0,002 % vollständig gehemmt (0-5 % Befall).

Beispiel 3.7: Wirkung gegen Erysiphae graminis auf Gerste

a) Residual-protektive Wirkung

Ca. 8 cm hohe Gerstenpflanzen werden mit einer auf Spritzpulver des Wirkstoffes hergestellten Spritzbrühe (0,006 % Aktivsubstanz) besprüht. Nach 3-4 Stunden werden die behandelten Pflanzen mit Konidien

des Pilzes bestäubt. Die infizierten Gerstenpflanzen werden in einem Gewächshaus bei ca. 22°C aufgestellt und der Pilzbefall nach 10 Tagen beurteilt.

b) Systemische Wirkung

Zu ca. 8 cm hohen Gerstenpflanzen wird eine aus Spritzpulver des Wirkstoffes hergestellte Spritzbrühe gegossen (0,002 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden werden die behandelten Pflanzen mit Konidien des Pilzes bestäubt. Die infizierten Gerstenpflanzen werden in einem Gewächshaus bei ca. 22°C aufgestellt und der Pilzbefall nach 10 Tagen beurteilt.

Die Pflanzen waren in beiden Versuchen befallsfrei, die Kontrollpflanzen vollständig befallen. Im Versuch a) wurde mit den Wirkstoffen Nr. 1, 2, 3, 4, 7, 8, 9, 17, 26, 27, 29, 30 und 33-35 wurde der Pilzbefall auch noch in einer Konzentration von 0,002 % vollständig gehemmt (0-5 % Befall).

Beispiel 3.8: Wirkung gegen Rhizoctonia solani (Bodenpilz auf Reispflanzen)

Protektiv-lokale Bodenapplikation

12 Tage alte Reispflanzen werden mit einer aus einer Formulierung des Wirkstoffes hergestellten Spritzbrühe (0,002 % Aktivsubstanz), ohne oberirdische Pflanzenteile zu kontaminieren, angegossen. Zur Infizierung der behandelten Pflanzen wird eine Suspension von Myzel und Sklerotien von R. solani auf die Bodenoberfläche gegeben. Nach 6-tägiger Inkubation bei 27°C (Tag), bzw. 23°C (Nacht) und 100 % rel. Luftfeuchtigkeit (Feuchtkasten) in der Klimakammer wird der Pilzbefall auf Blattscheide, Blättern und Stengel beurteilt.

Nach Behandlung mit dem Wirkstoff I trat fast kein Befall auf. Verbindung Nr. 7 verhinderte den Befall vollständig.

## Patentansprüche

1. Eine macrocyclische Verbindung der Formel I

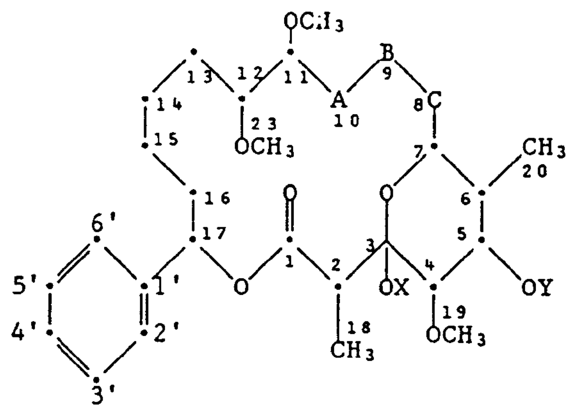

(I)

worin die Substituenten folgende Bedeutung haben:
X = Wasserstoff oder Methyl,
Y = Wasserstoff, -CHO, $-COCH_3$, Benzyl, Methyl oder eine Silyl-Schutzgruppe, und
A-B-C folgende $C_3$-Kettenglieder:

a) 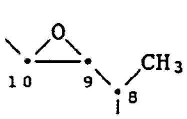 b) 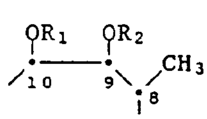 c) 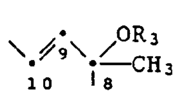 d) 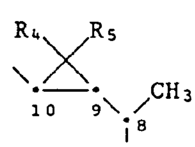

worin
$R_1$, $R_2$ und $R_3$ unabhängig voneinander Wasserstoff, Methyl, -CHO, $-COCH_3$, oder eine Silyl-Schutzgruppe darstellen, und
$R_4$ und $R_5$ Wasserstoff, Halogen oder -COOAlkyl ($C_1$-$C_3$) bedeuten.

2. Eine Verbindung ausgewählt aus
9,10-Epoxi-Soraphen A,
9,10-Dihydroxy-Soraphen A,
5,9,10-Triformyl-Soraphen A,
5,9,10-Triacetyl-Soraphen A, und
9,10-Epoxi-Soraphen A-5-acetat
gemäss Anspruch 1.

3. Verfahren zur Herstellung einer Verbindung der Formel I gemäss Anspruch 1 aus Soraphen A der Formel

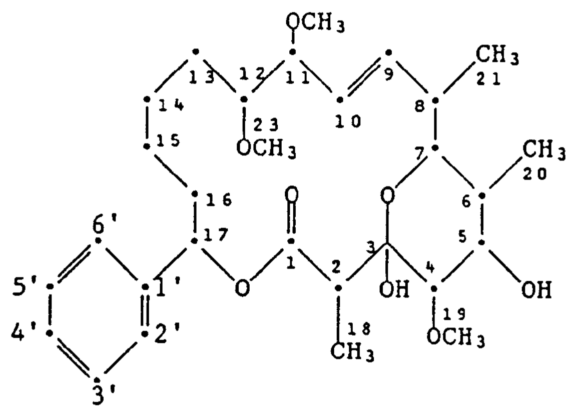

Soraphen A

gekennzeichnet wahlweise und unabhängig von der Reihenfolge
a) durch Carben-Anlagerung, Selendioxid-Oxidation, Osmiumtetroxid-Oxidation oder Epoxidierung in 9,10-Stellung unter gegebenenfalls weiterer Ring-Oeffnung des gebildeten Epoxids, und/oder, sofern gewünscht,
b) durch Methylierung der 3-OH-Gruppe, und/oder, sofern gewünscht,
c) durch Formylierung, Acetylierung, Methylierung, Benzylierung bzw. Silylierung der 5-OH-Gruppe und/oder, sofern gewünscht, der neu gebildeten OH-Gruppen in 8-, 9- oder 10-Stellung.

4. Verfahren gemäss Anspruch 3, dadurch gekennzeichnet, dass im Reaktionsschritt c) eine Formylierung, Acetylierung, Methylierung, Benzylierung bzw. Silylierung der 5-OH-Gruppe durchgeführt wird.

5. Mittel zur Bekämpfung und Verhütung von Pflanzenkrankheiten enthaltend als mindestens einen Wirkstoff eine Verbindung der Formel I gemäss Anspruch 1, zusammen mit einem geeigneten Trägermaterial.

6. Mittel gemäss Anspruch 5 enthaltend als Wirkstoff eine Verbindung gemäss Anspruch 2.

7. Verwendung der Verbindung der Formel I gemäss Anspruch 1 zur Bekämpfung von Pflanzenkrankheiten bzw. zur Verhütung von Krankheitsbefall.

## Claims

1. A macrocyclic compound of the formula I,

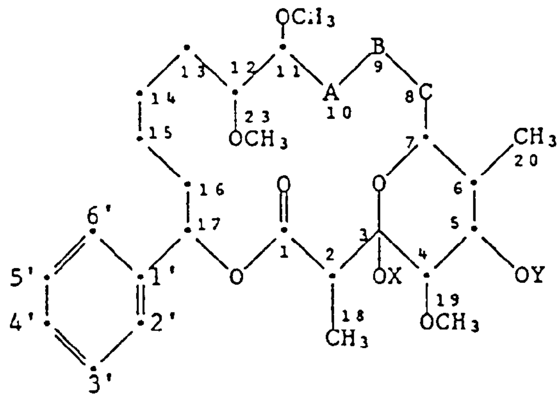

(I)

in which the substituents have the following meaning:
X is hydrogen or methyl,
Y is hydrogen, -CHO, $-COCH_3$, benzyl, methyl or a silyl protecting group, and
A-B-C is the following $C_3$-chain members:

a) 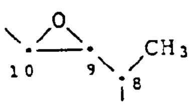 b) 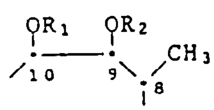 c) 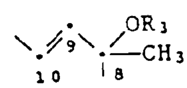 d) 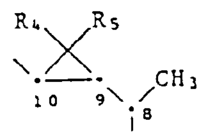

in which $R_1$, $R_2$ and $R_3$, independently of one another, are hydrogen, methyl, -CHO, $-COCH_3$, or a silyl protecting group, and $R_4$ and $R_5$ are hydrogen, halogen or $-COO(C_1-C_3)$alkyl.

2. A compound selected from
9,10-epoxy-soraphen A,
9,10-dihydroxy-soraphen A,
5,9,10-triformyl-soraphen A,
5,9,10-triacetyl-soraphen A,
9,10-epoxy-soraphen A 5-acetate,
according to claim 1.

3. A process for the preparation of a compound of the formula I according to claim 1 from soraphen A of the formula

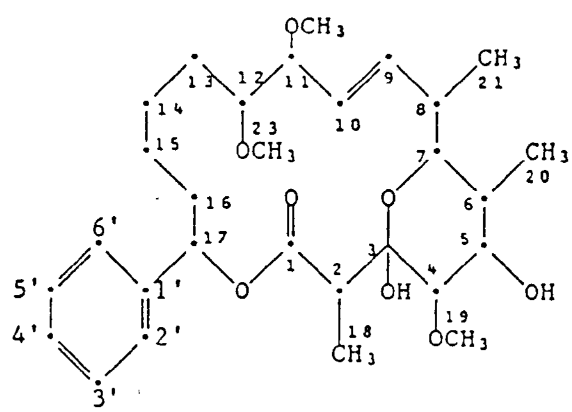

soraphen A

having the features, optionally and irrespective of the sequence,
a) carbene addition, selenium dioxide oxidation, osmium tetroxide oxidation or epoxidation in the 9,10-position, with or without further ring opening of the epoxide formed, and/or, if desired,
b) methylation of the 3-OH group, and/or, if desired,
c) formylation, acetylation, methylation, benzylation or silylation of the 5-OH group and/or, if desired,

of the newly formed OH groups in the 8-, 9- or 10-position.

**4.** A process according to claim 3, wherein a formylation, acetylation, methylation, benzylation or silylation of the 5-OH group is carried out in reaction step c).

**5.** An agent for controlling and preventing plant diseases, containing, as at least one active compound, a compound of the formula I according to claim 1, together with a suitable carrier material.

**6.** An agent according to claim 5, containing, as active compound, a compound according to claim 2.

**7.** The use of the compound of the formula I according to claim 1 for controlling plant diseases or for preventing disease infestation.

## Revendications

1.- Un composé macrocyclique de formule I :

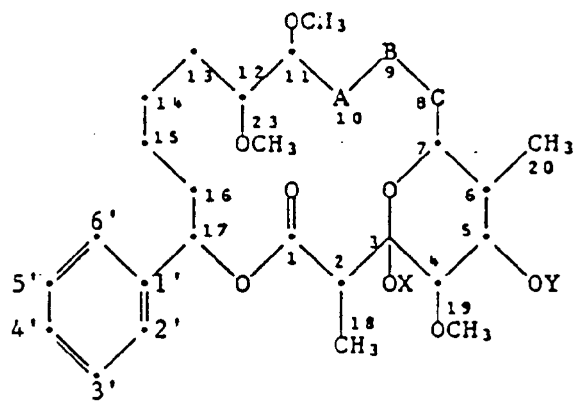

(I)

dans laquelle les symboles ont les significations suivantes:
X représente l'hydrogène ou un groupe méthyle,
Y représente l'hydrogène, un groupe -CHO, $-COCH_3$, benzyle, méthyle ou un groupe protecteur silyle et
A-B-C représente les chaînons en $C_3$ suivants :

a) 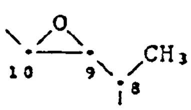 b) 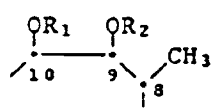 c) 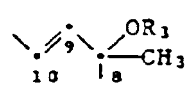 d) 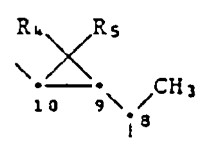

dans lesquels :
$R_1$, $R_2$ et $R_3$ représentent chacun, indépendamment les uns des autres, l'hydrogène, un groupe méthyle, -CHO, $-COCH_3$ ou un groupe protecteur silyle, et $R_4$ et $R_5$ représentent l'hydrogène, des halogènes ou des groupes -COO-alkyle en $C_1$-$C_3$.

2.- Un composé choisi parmi les suivants :
9,10-époxy-Soraphen A
9,10-dihydroxy-Soraphen A
5,9,10-triformyl-Soraphen A
5,9,10-triacétyl-Soraphen A, et
5-acétate du 9,10-époxy-Soraphen
selon la revendication 1.

3.- Procédé de préparation d'un composé de formule I de la revendication 1, à partir du Soraphen A de formule

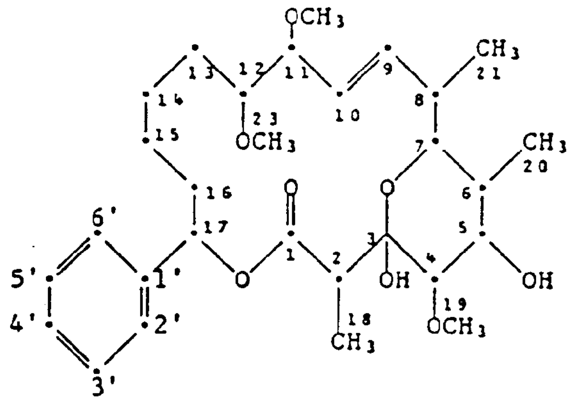

Soraphen A

caractérisé au choix et indépendamment de l'ordre observé:

(a) par la fixation par addition d'un carbène, l'oxydation par le dioxyde de sélénium, l'oxydation par le tétroxyde d'osmium ou l'époxydation en position 9, 10 avec le cas échéant l'ouverture du.cycle de l'époxyde formé, et/ou, si on le désire,

(b) par la méthylation du groupe 3-OH et/ou, si on le désire,

(c) par la formylation, l'acétylation, la méthylation, la benzylation ou la silylation du groupe 5-OH et/ou, si on le désire, des groupes OH nouvellement formés en position 8, 9 ou 10.

4.- Procédé selon la revendication 3, caractérisé en ce que, dans le stade de réaction (c), on procède à une formylation, une acétylation, une méthylation, une benzylation ou une silylation du groupe 5-OH.

5.- Produit pour prévenir ou traiter les maladies des végétaux, contenant au moins une substance active consistant en un composé de formule I de la revendication 1, avec un véhicule approprié.

6.- Produit selon la revendication 5, contenant en tant que substance active un composé selon la revendication 2.

7.- Utilisation du composé de formule I de la revendication 1 pour le traitement des maladies des végétaux ou pour la prévention des maladies.